# EUROPÄISCHE PATENTSCHRIFT

(11) **EP 0 745 588 B1**
(45) Veröffentlichungstag und Bekanntmachung des Hinweises auf die Patenterteilung: **11.03.1998**
(21) Anmeldenummer: 96107306.1
(22) Anmeldetag: 09.05.1996
(51) Int. Cl.: C07C 407/00, C07C 409/34, C08F 4/34

(54) **Vorübergehend inaktiviertes Benzoylperoxid, Verfahren zu seiner Herstellung und seine Verwendung**
Temporary inactivated benzoyl peroxide, process for its preparation and use thereof
Péroxyde de benzoyle inactivé temporairement, procédé pour sa préparation et son utilisation

(30) Priorität: 02.06.1995 DE 19520369
(43) Veröffentlichungstag der Anmeldung: 04.12.1996
(73) Patentinhaber: KOCH MARMORIT GmbH, 79283 Bollschweil (DE)
(72) Erfinder: Banhardt, Volker, Dr., 79282 Ballrechten-Dottingen (DE); Opferkuch, Roland, 79189 Bad Krozingen-Tunsel (DE)
(74) Vertreter: Werner, Hans-Karsten, Dr.Dipl.-Chem.

(56) Entgegenhaltungen:
- EP-A- 0 038 988
- BE-A- 522 868
- DD-A- 114 115
- DE-A- 3 900 752
- US-A- 4 255 277
- US-A- 5 276 202

## Beschreibung

Benzoylperoxid, gelegentlich auch Dibenzoylperoxid genannt, ist eine Substanz, die vielfach als Initiator für Radikal-Kettenreaktionen verwendet wird. Im Handel befindliches Benzoylperoxid enthält mindestens 20 % Wasser oder 50 % andere Phlegmatisierungsmittel in Pasten- oder Pulverform. Die BE-A-522868 beschreibt ein Verfahren, bei dem organische Peroxide, insbesondere Benzoylperoxid durch schrittweise Zugabe eines Plastifizierungsmittels getrocknet und phlegmatisiert werden. Das am häufigsten verwendete Phlegmatisierungsmittel ist Dimethylphtalat. Es können aber auch Öl, Paraffin, Wachs, Centralit® und andere Stabilisatoren eingesetzt werden. Man nimmt an, daß Phlegmatisierungsmittel als Wärmeübertragungsisolatoren wirken und somit die Wärmeleitung von einem Kristall zum anderen verhindern.

Benzoylperoxid wird beispielsweise eingesetzt als Initiator der Polymerisation und/oder Vernetzung von Styrol, Buna S, Methacrylaten, Allylestern und anderen ungesättigten Polyestern und Polyolefinen. Die radikalisch initiierte Polymerisation tritt im allgemeinen unverzüglich ein, sobald die polymerisierbaren Substanzen mit dem Benzoylperoxid in Berührung kommen. Gelegentlich wird aber aus technischen Gründen gefordert, daß die Polymerisation erst zu einem späteren Zeitpunkt eintritt. In solchen Fällen muß man das polymerisierbare Gemisch und den Initiator voneinander getrennt halten. Dies geschieht beispielsweise gemäß DD-A-114 115 durch Einschmelzen des polymerisierbaren Gemisches in einen Glaszylinder, der mit einem phlegmatisierten Härter umgeben ist, oder durch Einschmelzen des Initiators in Glasampullen und Einbettung in die polymerisierbare Substanz. Diese voneinander getrennten Gemische werden durch ein weiteres äußeres Gefäß ummantelt, welches ebenfalls zerstört werden muß und zu einer zusätzlichen Verunreinigung der Masse führt. Durch mechanische Einwirkung können dann die Glasgefäße und/oder die Kunststoffummantelung zum gewünschten Zeitpunkt zerbrochen werden, so daß der Initiator sich mit den polymerisierbaren Komponenten vermischen kann. Ein Nachteil dieser Methode ist, daß das fertig polymerisierte Gemisch durch Glasscherben und/oder Kunststoffteilchen verunreinigt ist. Es besteht somit das Bedürfnis, Benzoylperoxid vorübergehend zu inaktivieren, ohne dem Gemisch aus polymerisierbaren Substanzen und Initiator unnötig weitere Substanzen, Stoffe oder Verunreinigungen beimischen zu müssen.

Diese Aufgabe konnte jetzt überraschend einfach dadurch gelöst werden, daß mit Dimethylphtalat phlegmatisiertes Benzoylperoxid zu Tabletten verpreßt wird. Derartige Tabletten aus mit Dimethylphtalat phlegmatisierten Benzoylperoxid können überraschenderweise in die polymerisierbaren Gemische eingegeben werden, ohne daß die Polymerisation oder Vernetzung eintritt. Dieser Effekt kann nicht allein darauf zurückgeführt werden, daß durch die Tablettierung die Oberfläche des Benzoylperoxids verkleinert wird. Mit Calciumsulfatdihydrat (Gips) phlegmatisiertes Dimethylperoxid kann beispielsweise auf diesem Wege nicht vorübergehend inaktiviert werden. Eine nachträgliche Erklärung des überraschenden Befundes könnte darin bestehen, daß beim Tablettieren die Benzoylperoxid-Kristalle zumindest an der Oberfläche der Tablette mit einem durchgehenden dünnen Film von Dimethylphtalat überzogen werden, so daß das Benzoylperoxid nicht nur phlegmatisiert, sondern sogar völlig inaktiviert ist. Um die Polymerisation oder Vernetzung in Gang zu setzen, ist es erfindungsgemäß nur noch nötig, die Tablette zu zerstören. Die Zerstörung kann beispielsweise durch Zerdrücken, Zerreiben, Zerstampfen oder Bearbeitung mit einem starken Rührer erfolgen. Die im Inneren der Tablette vorhandenen Mengen an Benzoylperoxid bleiben somit zwar phlegmatisiert aber trotzdem voll aktiv für die Initiierung von Radikal-Kettenpolymerisationen.

Das erfindungsgemäß vorübergehend inaktivierte Benzoylperoxid kann somit hervorragend eingesetzt werden zu Polymerisations- oder Vernetzungsreaktionen, die erst zu einem bestimmten Zeitpunkt gewünscht sind. Ein Beispiel hierfür sind Ampullen, die mit dem polymerisierbaren Gemisch, Füllstoffen und einer oder mehreren erfindungsgemäß hergestellten Tabletten gefüllt sind und in Bohrlöcher eingesetzt werden können. Sobald der in dem Bohrloch einzusetzende Anker, Haken oder das sonstige Befestigungselement eingedreht oder eingeschlagen wird, zerbricht die Ampulle und auch die Tablette, so daß die Polymerisation in Gang gesetzt wird.

Andere Anwendungsgebiete sind flüssige oder zähpastöse Gemische aus polymerisierbaren Substanzen, gegebenenfalls Füllstoffen und den erfindungsgemäßen Tabletten, die eingesetzt werden sollen, Löcher oder Fugen zu verschließen. Auch hierbei muß nur dafür gesorgt werden, daß die erfindungsgemäßen Tabletten in den polymerisierbaren Substanzen zerschlagen und mehr oder weniger gleichmäßig verteilt werden. Es setzt dann mehr oder weniger schnell die Polymerisation ein, so daß es zur Aushärtung des Gemisches kommt.

Das Verfahren zur Tablettierung kann in üblichen Tablettenpressen durchgeführt werden. Geeignet sind Pressen für glatte Tabletten oder auch konkavgewölbte Tabletten. Prinzipiell sind auch andere Tablettenformen denkbar wie Tabletten mit einer Bruchkerbe. Diese länglichen Tabletten können gegebenenfalls aus räumlichen Gründen Vorteile haben gegenüber üblichen runden Tabletten.

Ein besonderer Vorteil der erfindungsgemäßen Tabletten ist, daß keine weiteren Hilfsmittel nötig sind. Prinzipiell ist es aber durchaus möglich, den Tabletten inerte Zusätze beizumischen, die die Stabilität der Tabletten nicht beeinflußen, jedoch dem endgültig polymerisierten Gemisch zusätzlich gewünschte Eigenschaften verleihen. So lassen sich diesen Tabletten auch Farbstoffe oder Farbpigmente beimischen, die das fertig polymerisierte Gemisch einfärben. Eine gleichmäßige Färbung des Gemisches ist gleichzeitig ein Anzeichen dafür, daß das Benzoylperoxid ausreichend gleichmäßig in der polymerisierbaren Mischung verteilt worden ist.

Gegenstand der vorliegenden Erfindung ist somit zunächst das vorübergehend inaktivierte Benzoylperoxid bestehend aus zu Tabletten verpreßten Benzoylperoxid, welches mit Dimethylphtalat phlegmatisiert ist. Ein weiterer Gegenstand der Erfindung ist das Verfahren zur vorübergehenden Inaktivierung von Benzoylperoxid, das dadurch gekennzeichnet ist, daß ein mit Dimethylphtalat phlegmatisiertes Benzoylperoxid zu Tabletten verpreßt wird. Schließlich ist Gegenstand der vorliegenden Erfindung die Verwendung von vorübergehend inaktiviertem Benzoylperoxid in Form von Tabletten aus Benzoylperoxid, welches mit Dimethylphtalat phlegmatisiert ist zur Herstellung von radikalisch polymerisierbaren und/oder vernetzbaren Gemischen.

## Patentansprüche

1. Vorübergehend inaktiviertes Benzoylperoxid bestehend aus zu Tabletten verpreßten Benzoylperoxid, welches mit Dimethylphtalat phlegmatisiert ist.

2. Verfahren zur vorübergehenden Inaktivierung von Benzoylperoxid, dadurch gekennzeichnet, daß ein mit Dimethylphtalat phlegmatisiertes Benzoylperoxid zu Tabletten verpreßt wird.

3. Verwendung von vorübergehend inaktiviertem Benzoylperoxid in Form von Tabletten aus Benzoylperoxid, welches mit Dimethylphtalat phlegmatisiert ist zur Herstellung von radikalisch polymerisierten und/oder vernetzten Gemischen eines oder mehrerer radikalisch polymerisierbarer und/oder vernetzbarer Monomere.

## Claims

1. A temporarily inactivated benzoyl peroxide comprising benzoyl peroxide compressed to tablets being desensitized by dimethyl phthalate.

2. A process for the temporary inactivation of benzoyl peroxide, characterized in that a benzoyl peroxide desensitized by dimethyl phthalate is compressed to tablets.

3. The use of temporarily inactivated benzoyl peroxide in the form of tablets of benzoyl peroxide being desensitized by dimethyl phthalate for the production of free-radical polymerizable and/or crosslinkable mixtures of free-radical polymerizable and/or crosslinkable monomers.

## Revendications

1. Peroxyde de benzoyle temporairement inactivé, formé de peroxyde de benzoyle pressé en comprimés, qui est stabilisé avec du phtalate de diméthyle.

2. Procédé d'inactivation temporaire de peroxyde de benzoyle, caractérisé en ce qu'un peroxyde de benzoyle stabilisé avec du phtalate de diméthyle est pressé en comprimés.

3. Utilisation de peroxyde de benzoyle temporairement inactivé, sous la forme de comprimés de peroxyde de benzoyle, qui est stabilisé avec du phtalate de diméthyle, pour la préparation de mélanges, polymérisés par polymérisation radicalaire et/ou réticulés, d'un ou plusieurs monomères polymérisables par des radicaux libres et/ou réticulables.
